# EUROPEAN PATENT SPECIFICATION

(11) **EP 1 562 495 B1**
(45) Date of publication and mention of the grant of the patent: **04.11.2009**
(21) Application number: 03756886.2
(22) Date of filing: 24.09.2003
(51) Int. Cl.: A61B 17/32

(54) **ULTRASONIC SURGICAL INSTRUMENT HAVING AN INCREASED WORKING LENGTH**
CHIRURGISCHES ULTRASCHALLINSTRUMENT MIT ERHÖHTER ARBEITSLÄNGE
INSTRUMENT CHIRURGICAL ULTRASONORE A LONGUEUR D'INTERVENTION ACCRUE

(30) Priority: 24.09.2002 US 413120 P
(43) Date of publication of application: 17.08.2005
(73) Proprietor: ETHICON ENDO-SURGERY, INC., Cincinnati, Ohio 45242 (US)
(72) Inventor: BEAUPRE, Jean, Cincinnati, OH 45249 (US)
(74) Representative: Tunstall, Christopher Stephen
(86) International application number: PCT/US2003/030601
(87) International publication number: WO 2004/028349

(56) References cited:
- US-A- 5 322 055
- US-B1- 6 193 709
- US-B1- 6 340 352
- US-B2- 6 309 400

## Description

### Field of the Invention

The present invention relates to an ultrasonic surgical instrument for cutting, coagulating, grasping and blunt-dissecting tissue, and particularly relates to an ultrasonic surgical instrument having longer working lengths. The present invention is, in one embodiment, specifically adapted for endoscopic surgery, although it has other surgical applications as well.

### Background of the Invention

Ultrasonic instruments, including both hollow core and solid core instruments, are used for the safe and effective treatment of many medical conditions. Ultrasonic instruments, and particularly solid core ultrasonic instruments, are advantageous because they may be used to cut and/or coagulate organic tissue using energy in the form of mechanical vibrations transmitted to a surgical end-effector at ultrasonic frequencies. Ultrasonic vibrations, when transmitted to organic tissue at suitable energy levels and using a suitable end-effector, may be used to cut, dissect, or cauterize tissue. Ultrasonic instruments utilizing solid core technology are particularly advantageous because of the amount of ultrasonic energy that may be transmitted from the ultrasonic transducer through the waveguide to the surgical end-effector. Such instruments are particularly suited for use in minimally invasive procedures, such as endoscopic or laparoscopic procedures, wherein the end-effector is passed through a trocar to reach the surgical site.

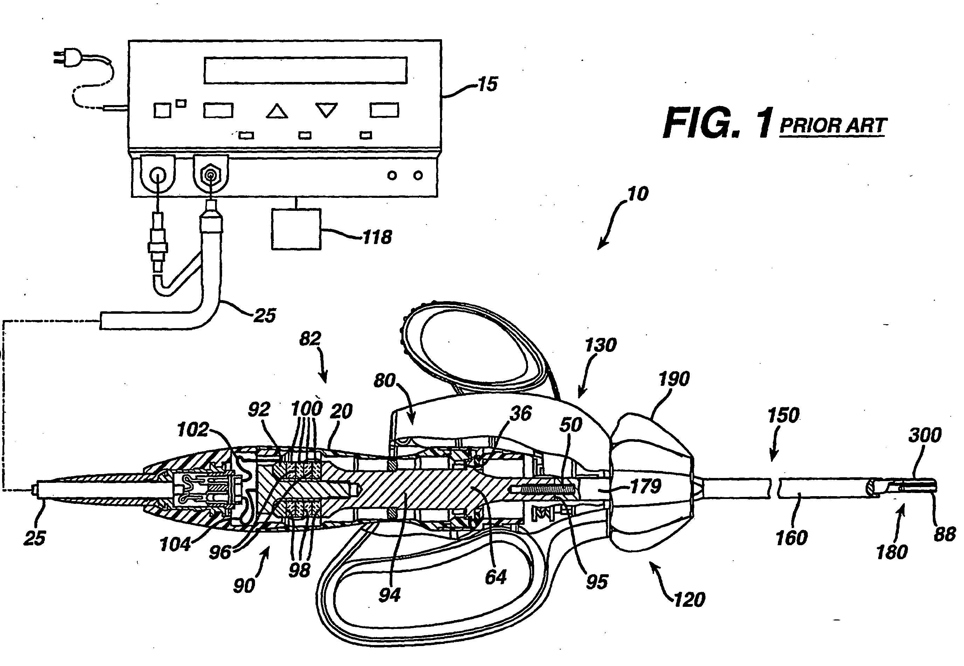

Figure 1 illustrates an exemplary ultrasonic system 10 comprising an ultrasonic signal generator 15 with ultrasonic transducer 82, hand piece housing 20, and clamp coagulator 120 in accordance with the present invention. Clamp coagulator 120 may be used for open or laparoscopic surgery. The ultrasonic transducer 82, which is known as a "Langevin stack", generally includes a transduction portion 90, a first resonator or end-bell 92, and a second resonator or fore-bell 94, and ancillary components. The ultrasonic transducer 82 is preferably an integral number of one-half system wavelengths (nλ/2) in length as will be described in more detail later. An acoustic assembly 80 includes the ultrasonic transducer 82, mount 36, velocity transformer 64 and surface 95.

The distal end of end-bell 92 is connected to the proximal end of transduction portion 90, and the proximal end of fore-bell 94 is connected to the distal end of transduction portion 90. Fore-bell 94 and end-bell 92 have a length determined by a number of variables, including the thickness of the transduction portion 90, the density and modulus of elasticity of the material used to manufacture end-bell 92 and fore-bell 94, and the resonant frequency of the ultrasonic transducer 82. The fore-bell 94 may be tapered inwardly from its proximal end to its distal end to amplify the ultrasonic vibration amplitude as velocity transformer 64, or alternately may have no amplification.

The piezoelectric elements 100 may be fabricated from any suitable material, such as, for example, lead zirconate-titanate, lead meta-niobate, lead titanate, or other piezoelectric crystal material. Each of the positive electrodes 96, negative electrodes 98, and piezoelectric elements 100 has a bore extending through the center. The positive and negative electrodes 96 and 98 are electrically coupled to wires 102 and 104, respectively. Wires 102 and 104 are encased within cable 25 and electrically connectable to ultrasonic signal generator 15 of ultrasonic system 10.

Wires 102 and 104 transmit the electrical signal from the ultrasonic signal generator 15 to positive electrodes 96 and negative electrodes 98. The piezoelectric elements 100 are energized by an electrical signal supplied from the ultrasonic signal generator 15 in response to a foot switch 118 to produce an acoustic standing wave in the acoustic assembly 80. The electrical signal causes disturbances in the piezoelectric elements 100 in the form of repeated small displacements resulting in large compression forces within the material. The repeated small displacements cause the piezoelectric elements 100 to expand and contract in a continuous manner along the axis of the voltage gradient, producing longitudinal waves of ultrasonic energy.

An ultrasonic transmission 80 is generally defined as a waveguide 179, an end effector 88 and an ultrasonic transducer 82. Further, the ultrasonic waveguide 179 and end effector 88 are, in combination, referred to as a "blade". Ultrasonic transducer 82 converts the electrical signal from ultrasonic signal generator 15 into mechanical energy that results in primarily longitudinal vibratory motion of the ultrasonic transducer 82, waveguide 179 and end-effector 88 at ultrasonic frequencies. Ultrasonic end effector 88 and ultrasonic transmission waveguide 179 are illustrated as a single unit construction from a material suitable for transmission of ultrasonic energy such as, for example, Ti6Al4V (an alloy of titanium including aluminum and vanadium), aluminum, stainless steel, or other known materials. Alternately, end effector 88 may be separable (and of differing composition) from waveguide 179, and coupled by, for example, a stud, welding, gluing, or other known methods.

When the acoustic assembly 80 is energized, a vibratory motion standing wave is generated through the acoustic assembly 80. The amplitude of the vibratory motion at any point along the acoustic assembly 80 depends on the location along the acoustic assembly 80 at which the vibratory motion is measured. A minimum or zero crossing in the vibratory motion standing wave is generally referred to as a node (i.e., where motion is usually minimal), and an absolute value maximum or peak in the standing wave is generally referred to as an anti-node. The distance between an anti-node and its nearest node is one-quarter wavelength (λ /4).

In order for the acoustic assembly 80 to deliver energy to end-effector 180, all components of acoustic assembly 80 must be acoustically coupled to the ultrasonically active portions of clamp coagulator 120. The distal end of the ultrasonic transducer 82 may be acoustically coupled at surface 95 to the proximal end of an ultrasonic waveguide 179 by a threaded connection such as stud 50.

The components of the acoustic assembly 80 are preferably acoustically tuned such that the length of any assembly is an integral number of one-half wavelengths (nλ /2), where the wavelength λ is the wavelength of a pre-selected or operating longitudinal vibration drive frequency f_{d} of the acoustic assembly 80, and where n is any positive integer. It is also contemplated that the acoustic assembly 80 may incorporate any suitable arrangement of acoustic elements.

The clamp coagulator 120 may include an instrument housing 130, and an elongated member 150. The elongated member 150 can be selectively rotated with respect to the instrument housing 130. Located at the distal end of the outer tube 160 is an clamp element 180, which comprises the end effector 88 and clamp arm 300 for performing various tasks, such as, for example, grasping tissue, cutting tissue and the like.

The ultrasonic waveguide 179 of the elongated member 150 extends through an aperture of an inner tube. The ultrasonic waveguide 179 is preferably substantially semi-flexible. It will be recognized that the ultrasonic waveguide 179 may be substantially rigid or may be a flexible wire. Ultrasonic vibrations are transmitted along the ultrasonic waveguide 179 in a longitudinal direction to vibrate the ultrasonic end effector 88.

The ultrasonic waveguide 179 may, for example, have a length substantially equal to an integral number of one-half system wavelengths (nλ/2). The ultrasonic waveguide 179 may be preferably fabricated from a solid core shaft constructed out of material that propagates ultrasonic energy efficiently, such as titanium alloy (i.e., Ti-6Al-4V) or an aluminum alloy. The ultrasonic waveguide 179 may also amplify the mechanical vibrations transmitted to the ultrasonic end effector 88 as is well known in the art.

The ultrasonic end effector 88 may have a length substantially equal to an integral multiple of one-half system wavelengths (nλ/2). The distal end of ultrasonic end effector 88 may be disposed near an antinode in order to provide the maximum longitudinal excursion of the distal end. When the transducer assembly is energized, the distal end of the ultrasonic end effector 88 is configured to move in the range of, for example, approximately 10 to 500 microns peak-to-peak, and preferably in the range of about 30 to 150 microns at a predetermined vibrational frequency.

Ultrasonic generators, such as the model number GEN01, from Ethicon Endo-Surgery, Inc., Cincinnati, Ohio, can lock onto any longitudinal frequency between 51 and 57.5 kHz. Ultrasonic end effectors are designed to have only one resonance in this range. Presently, ultrasonic blades are limited to a working length of about 36cm, though a need has arisen for end effectors having a working length of 40-45cm in order to perform applications requiring additional length. The addition of ½ waves in an ultrasonic transmission assembly incurs the penalty of having mode shape frequencies closer together. At some point, the mode shape frequencies are so close together that two or more will be within the lock range of a generator/transducer. Each half wave of Ti6A4V is currently limited to about 43.18mm (about 1.7 inches) long unless the cross section is modified. Presently, the ultrasonic generators in use are not compatible with end effectors having more than 9 (½ wave) sections, thereby limiting the working length of a titanium end effector to 39.116cm (15.4 inches) or 39cm. A further ultrasound treatment system is disclosed in US patent 6340352, including a handpiece and a longitudinal probe. An end-effector control rod is attached to the probe by means of couplers connected to the probe at locations spaced along the length of the probe. The couplers are placed in ditches formed on the probe at positions corresponding to wave nodes.

The present invention addresses the deficiencies of the prior art.

### Brief Summary of the Invention

The present invention provides the operator with an ultrasonic device having a long working length for use in applications where this feature is desired, such as in the field of bariatrics, without adding ½ wave segments and yet providing the generator the same effective modes to lock onto. The present invention also provides for a reduction in the overall length of an ultrasonic waveguide, which may be beneficial for applications where a shorter waveguide is desirable. The present invention provides for a blade having altered cross sectional areas and/or stiffness of ½ wave segments of the waveguide and/or end effector. The ½ wave segments of the waveguide or end effector comprise a number of geometries and function to extend or decrease the length of a waveguide and/or end effector without adding or removing ½ wave segments. The present invention is intended to function with conventional ultrasonic transducers at conventional frequencies.

It would be advantageous to provide an ultrasonic surgical instrument with a longer working length that does not require the addition of ½ wave segments. It would be further advantageous to provide an end effector with a longer working length that is simple to manufacture, thereby reducing both production and patient costs. It would also be advantageous to provide an ultrasonic instrument with an extended work length that is compatible with generators presently available. It would be even further advantageous to provide a means of reducing the overall length of a waveguide without having to remove ½ wave segments, for applications where a shorter wavelength is desirable.

A further advantage of the present invention is that it provides serial amplification/deamplification. If a series of extended ½ waves are joined, and the nodes at resonance are biased to one side, each ½ wave will act as an amplifier or deamplifier. As a portion of a end effector warms up, frequency and node bias will change. This changes the serial amplification/deamplification, whereby functioning to decrease net amplification and net heat and creating a feedback loop. The feedback loop functions to maintain end effector temperature below a designated point intrinsic in the design of the end effector.

A still further advantage of the present invention comprises multi-mode resonance. Serial expanded ½ waves will maintain the same longitudinal frequency N, but N-1 and N+1 will decrease. This is of no concern in regards to N-1, but N+1 will converge on N, thereby initiating a multi-mode resonance. However, most of the nodes for N and N+1 are close to each other. The one exception is where N's node is N+1's anti-node surrounded by 2 nodes. Furthermore, the expanded ½ waves up to that point act as deamplifiers and afterwards as amplifiers. Therefore, the 90 degree out of phase anti-node tends to have low amplitude, resulting in a end effector (or waveguide) that can run at two frequencies with low impedence and low heat generation at the same time. It is also possible to create a device with the two mode shapes running at the same frequency.

The restriction is that the two mode shapes will be in phase at one end, and 180 degrees out of phase at the other end. If the two modes are at the same frequency, in phase at one end, out of phase and with equal amplitude at the other end, the canceled end can be extended by adding uniform diameter rods, maintaining both modes out of phase, superimposed. As many ½ waves can be added as desired.

Finally, if an equivalent system is joined to the one described above, it will reconvert the canceling waves into reinforcing waves. The result is a very long, thin, ultrasonic waveguide with zero motion over the bulk of the length. It may be possible to use a thin, flexible wire over this null zone to effectively guide ultrasonic energy from outside the body, through an uninsulated flexible catheter to a working end effector.

The present invention is useful in for endoscopic and open surgical applications. It is also useful for robotic-assisted surgery applications. An ultrasonic instrument according to the present invention is defined in appended claim 1 and in appended claim 2. Further optional features thereof are defined in subclaims 3-5.

### Brief Description of the Figures

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

FIGURE 1 is a partial cut-away elevation view of a representative ultrasonic surgical instrument of the prior art;

FIGURE 2 is a partial elevation view of a waveguide (not claimed) having two different cross- sectional areas;

FIGURE 2a is a partial elevation view of an alternate embodiment of a waveguide in Fig. 2 having at least two different cross-sectional areas;

FIGURE 3 is a partial elevation view of an alternate embodiment (not claimed) of a waveguide having two different cross-sectional areas; and

FIGURE 3a is a partial elevation view of an alternate embodiment of a waveguide in Fig. 3 having at least two different cross-sectional areas.

### Detailed Description of the Invention

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.
The invention is defined in claims 1 and 2. Preferred embodiments thereof are defined in dependent claims 3-5.

It is also understood that any one or more of the following-described embodiments, expressions of embodiments, examples, methods, etc. can be combined with any one or more of the other following-described embodiments, expressions of embodiments, examples, methods, etc.

The present invention is useful in combination with an end effector only, an end effector and a clamp, a shear configuration, or numerous other end-effectors. Examples of ultrasonic surgical instruments are disclosed in United States Patent nos. 5,322, 055 and 5,954, 736 and in combination with ultrasonic end effectors and surgical instruments as, for example, disclosed in United States Patent nos. 6,309, 400 B2, 6,283,981B1, and 6,325, 811 B1.

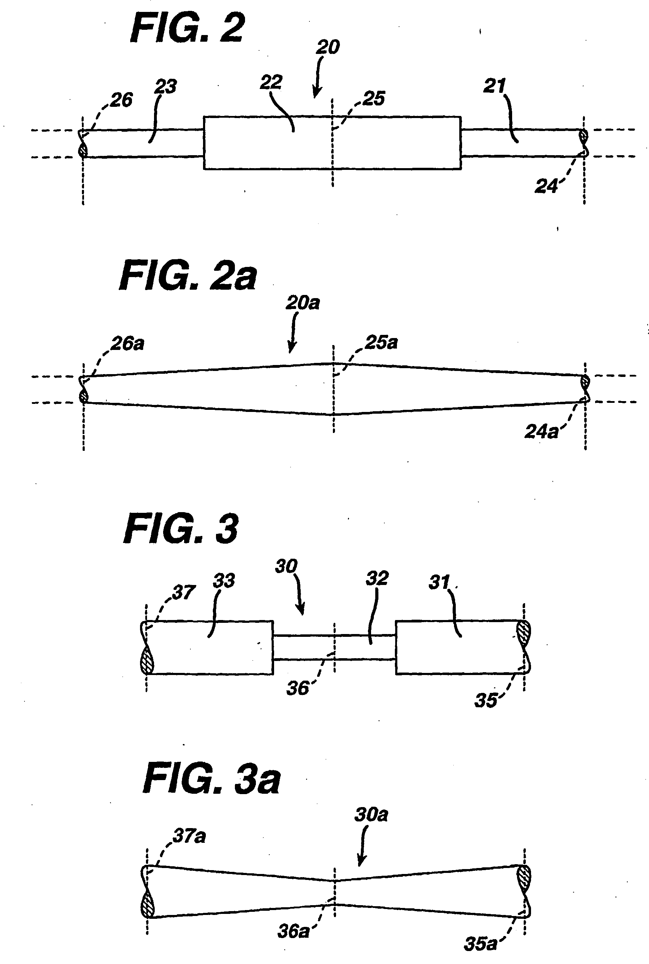

Fig. 2 illustrates a ½ wave segment 20 having a proximal reduced cross section segment 21, a central segment 22, and a distal reduced cross section segment 23. ½ wave segment 20 is part of an ultrasonic transmission assembly comprising a waveguide, an end effector, and an ultrasonic transducer as previously described. Fig. 2 further illustrates a first anti- node 24, a node 25, and a second anti-node 26, wherein at a standard frequency, the proximal most portion of proximal reduced cross section segment 21 is substantially aligned with first anti-node 24, the central symmetry line of central segment 22 is substantially aligned with node 25, and the distal most portion of distal segment 23 is substantially aligned with second anti-node 26. The segment distal to antinode 24 may have the same or different cross section than segment 21. Additionally, the segment proximal to antinode 26 may have the same or different cross section than segment 23.

Such cross section reductions may be applied on the distal portion of the ½ wave 20 or the proximal portion of the ½ wave 20 only, but the effect of lengthening ½ wave 20 will be reduced by a corresponding amount. A cross section increase of substantially short length in segment 21 or 23 will reduce the effect of lengthening the ½ wave 20, but can still be incorporated without eliminating the effect.

One related embodiment excited at a conventional frequency of 55kHz comprises an overall ½ wave segment 20 length of 61.3918mm (2.417 inches), a central segment 22 diameter of 3.556mm (0.140 inches), a proximal and distal segment 21 and 23 having a length of 14.859mm (0.585 inches), and a proximal and distal segment 21 and 23 having a diameter of 1.778mm (0.070 inches). This design of ½ wave segment 20 extends the length of the ½ wave segment 20 to 61.3918mm (2.417 inches), as opposed to a ½ wave having no cross sectional or stiffness variation, which is limited to about 43.18mm (1.7 inches) at that frequency when composed of the same material. The present invention contemplates combining ½ wave segment 20 with other ½ wave segments that are substantially the same as ½ wave segment 20, although other ½ wave segments may proximally begin, at an anti-node and end distally at a node. The present invention contemplates the use of a number of variations in cross sectional dimension that may be used to extend the length of ½ wave segment 20.

Stiffness and density may be used in place of cross-sectional variation to achieve a similar lengthening effect as above, wherein stiffness is increased in the range of central segment 22, and/or density is decreased in the range of proximal and distal tapered segments 21 and 23. This could be accomplished through various means including, but not limited to, increasing stiffness by local heat treatment, adding high modulus ceramic particles such as boron carbide, or using another alloy such as an iron or cobalt based alloy and decreasing density by using another alloy such as aluminum or adding ceramic particles such as boron carbide.

The combination of ½ wave segment 20 with other ½ wave segments having substantially the same features of ½ wave segment 20 functions to extend the length of the waveguide and/or end effector resulting in greater overall working length than that achieved by instruments having ½ wave segments with no cross- sectional area variation or stiffness variation.

Referring to Fig. 2a, in accordance with the invention, a plurality of such cross section reductions may be used, with smaller cross sections preferentially from node 25a towards antinodes 26a and 24a. This leads to a tapered shape from node 25a to antinodes 26a and 24a. One representative embodiment of the present invention excited at a conventional frequency of 55kHz comprises an overall ½ wave segment 20a length of 56.2356mm (2.214 inches), having a diameter of 3.556mm (0.140 inches) at node 25a and a diameter of 1.778mm (0.070 inches) at antinodes 24a and 26a. This design of ½ wave segment 20a extends the length of the 14 wave segment 20 to 56.2356mm (2.214 inches), as opposed to a ½ wave having no cross sectional or stiffness variation, which is limited to about 43.18mm (1.7 inches) at that frequency when composed of the same material. The present invention contemplates combining ½ wave segment 20a with other ½ wave segments that are substantially the same as ½ wave segment 20a or 20, although other ½ wave segments may proximally begin at an anti-node and end distally at a node. Further, the present invention contemplates the use of a number of variations in cross sectional dimension that may be used to extend the length of ½ wave segment 20a.

Fig. 3 illustrates an alternate embodiment comprising a ½ wave segment 30, wherein ½ wave segment 30 further comprises a first segment 31, a central segment 32, and a second segment 33, wherein first segment 31 is, at a normal operating frequency (55.5 kHz), substantially aligned with first anti-node 35, central segment 32 is substantially aligned with node 36, and second segment 33 is substantially aligned with second anti-node 37. First segment 31 and second segment 33 comprise a larger cross sectional area than central segment 32. The measurements of first segment 31, central segment 32, and second segment 33, comprise a number of variations in order to facilitate a reduction in the overall length of ½ wave segment 30.

In one embodiment, the measurement parameters of ½ wave segment 30 are designed in such a way as to function with a conventional ultrasonic transducer at a conventional frequency (55.5 kHz). Overall length of ½ wave segment 30 is 25.2222mm (0.993 inches), a central segment 32 diameter of 1.778mm (0.070 inches) and length 12.573mm (0.495 inches), proximal and distal segments 31 and 33 having a length of 0.63246mm (0.249 inches) and a diameter of 3.556mm (0.140 inches). ½ wave segment 30 may be attached to a number of other ½ wave segments having similar measurements substantially similar to ½ wave segment 30. The change in cross sectional area of ½ wave segment 30 functions to reduce the overall length of ½ wave segment 30, thereby reducing the overall length of the waveguide. The shortened waveguide is useful for procedures in which a shorter waveguide is beneficial. The segment distal to antinode 35 may have the same or different cross section than segment 31. Additionally, the segment proximal to antinode 37 may have the same or different cross section than segment 33.

Such larger cross sections may be applied on the distal portion ½ wave 30 or the proximal portion of the ½ wave 30 only, but the effect of shortening ½ wave 30 will be reduced by a corresponding amount. A cross section decrease of substantially short length in segment 31 or 33 will reduce the effect of lengthening the ½ wave 30, but can still be incorporated without eliminating the effect.

Referring to Fig. 3a, according to the invention, a plurality of such cross section reductions may be used, with smaller cross sections preferentially from antinodes 35a and 37a towards node 36a. In the extreme, this leads to a tapered shape. One representative embodiment of the present invention excited at a conventional frequency of 55kHz comprises an overall ½ wave segment 30a length of 32.3342mm (1.273 inches), having a diameter of 1.778mm (0.070 inches) at node 36a and a diameter of 3.556mm (0.140 inches) at antinodes 35a and 37a.

While preferred embodiments of the present invention have been shown and described herein as well as related embodiments, it will be obvious to those skilled in the art that such embodiments are provided by way of example only. In addition, it should be understood that every structure described above has a function and such structure can be referred to as a means for performing that function. Numerous variations, changes, and substitutions will now occur to those skilled in the art without departing from the invention. Accordingly, it is intended that the invention be limited only by the scope of the appended claims.

## Claims

1. An ultrasonic surgical instrument comprising:
a) an ultrasonic blade having a half-wave segment (20a), wherein said half-wave segment (20a) comprises a proximal segment substantially aligned with a proximal antinode (24a), a distal segment substantially aligned with a distal antinode (26a), and a central segment substantially aligned with a central node (25a), **characterized by** said central segment having a cross-sectional area greater than a cross-sectional area of said proximal and distal segments, and wherein said half-wave segment (20a) has a tapered shape from said node (25a) to said proximal and distal antinodes (24a, 26a).

2. An ultrasonic surgical instrument comprising:
a) an ultrasonic blade having a half-wave segment (30a), wherein said half-wave segment (30a) comprises a proximal segment substantially aligned with a proximal antinode (35a), a distal segment substantially aligned with a distal antinode (37a), and a central segment substantially aligned with a central node (36a), said proximal and distal segments having a cross-sectional area greater than a cross-sectional area of said central segment, **characterized by** said half-wave segment (30a) having a tapered shape from said node (36a) to said proximal and distal antinodes (35a, 37a).

3. An ultrasonic surgical instrument according to claim 1 or claim 2, comprising:
a) a housing;
b) a tubular sheath having a proximal end joined to the housing, and a distal end;
c) said ultrasonic blade being in the form of an ultrasonic waveguide positioned within the tubular sheath and having an end effector extending distally of the distal end of the tubular sheath.

4. The ultrasonic surgical instrument of claim 3 further comprising a clamp arm pivotally mounted on the distal end of the tubular sheath for pivotal movement with respect to the end effector for clamping tissue between the clamp arm and end effector.

5. The ultrasonic surgical instrument of any previous claim, wherein said half wave segment (30a) is combined with other half wave segments which are substantially the same.

## Patentansprüche

1. Mit Ultraschall arbeitendes chirurgisches Instrument, das aufweist:
a) eine Ultraschallklinge mit einem Halbwellensegment (20a), wobei das Halbwellensegment (20a) ein proximales Element, das im Wesentlichen mit einem proximalen Schwingungsbauch (24a) ausgerichtet ist, ein distales Segment, das im Wesentlichen mit einem distalen Schwingungsbauch (26a) ausgerichtet ist, und ein mittleres Segment, das im Wesentlichen mit einem mittig liegenden Schwingungsknoten (25a) ausgerichtet ist, aufweist, **dadurch gekennzeichnet, dass** das mittlere Segment eine Querschnittsfläche hat, die größer ist als eine Querschnittsfläche des proximalen und des distalen Segments und wobei das Halbwellensegment (20a) eine abgeschrägte Form von dem Schwingungsknoten (25a) zu dem proximalen und dem distalen Schwingungsbauch (24a, 26a) hat.

2. Mit Ultraschall arbeitendes chirurgisches Instrument, das aufweist:
a) eine Ultraschallklinge mit einem Halbwellensegment (30a), wobei das Halbwellensegment (30a) ein proximales Element, das im Wesentlichen mit einem proximalen Schwingungsbauch (35a) ausgerichtet ist, ein distales Segment, das im Wesentlichen mit einem distalen Schwingungsbauch (37a) ausgerichtet ist, und ein mittleres Segment, das im Wesentlichen mit einem mittig liegenden Schwingungsknoten (36a) ausgerichtet ist, aufweist, **dadurch gekennzeichnet, dass** das mittlere Segment eine Querschnittsfläche hat, die größer ist als eine Querschnittsfläche des proximalen und des distalen Segments und wobei das Halbwellensegment (30a) eine abgeschrägte Form von dem Schwingungsknoten (36a) zu dem proximalen und dem distalen Schwingungsbauch (35a, 37a) hat.

3. Mit Ultraschall arbeitendes chirurgisches Instrument nach Anspruch 1 oder Anspruch 2, das aufweist:
a) ein Gehäuse;
b) eine rohrförmige Hülse mit einem proximalen Ende, das mit dem Gehäuse verbunden ist, und einem distalen Ende;
c) wobei die Ultraschallklinge in der Form eines Ultraschall-Wellenleiters vorliegt, der innerhalb der rohrförmigen Hülse angeordnet ist und einen End-Effektor hat, der sich distal vom distalen Ende der rohrförmigen Hülse erstreckt.

4. Mit Ultraschall arbeitendes chirurgisches Instrument nach Anspruch 3, weiter mit einem Klemmarm, der schwenkbar an dem distalen Ende der rohrförmigen Hülse für die Schwenkbewegung in Bezug auf den End-Effektor angebracht ist, um Gewebe zwischen dem Klemmarm und dem End-Effektor einzuklemmen.

5. Mit Ultraschall arbeitendes chirurgisches Instrument nach einem vorangehenden Anspruch, bei dem das Halbwellensegment (30a) mit weiteren Halbwellensegmenten kombiniert ist, die im Wesentlichen die gleichen sind.

## Revendications

1. Instrument chirurgical ultrasonore comprenant :
a) une lame ultrasonore qui présente un segment demi-onde (20a), dans lequel ledit segment demi-onde (20a) comprend un segment proximal sensiblement aligné avec un ventre proximal (24a), un segment distal sensiblement aligné avec un ventre distal (26a), et un segment central sensiblement aligné avec un noeud central (25a), **caractérisé en ce que** ledit segment central présente une section transversale plus grande qu'une section transversale desdits segments proximal et distal, et dans lequel ledit segment demi-onde (20a) présente une forme conique à partir dudit noeud (25a) vers lesdits ventres proximal et distal (24a, 26a).

2. Instrument chirurgical ultrasonore comprenant :
a) une lame ultrasonore qui présente un segment demi-onde (30a), dans lequel ledit segment demi-onde (30a) comprend un segment proximal sensiblement aligné avec un ventre proximal (35a), un segment distal sensiblement aligné avec un ventre distal (37a), et un segment central sensiblement aligné avec un noeud central (36a), lesdits segments proximal et distal présentant une section transversale plus grande qu'une section transversale dudit segment central, **caractérisé en ce que** ledit segment demi-onde (30a) présente une forme conique à partir dudit du noeud (36a) vers lesdits ventres proximal et distal (35a, 37a).

3. Instrument chirurgical ultrasonore selon la revendication 1 ou la revendication 2, comprenant :
a) un logement ;
b) une gaine tubulaire qui présente une extrémité proximale jointe au logement, et une extrémité distale ;
c) ladite lame ultrasonore se présentant sous la forme d'un guide d'ondes ultrasonores positionné à l'intérieur de la gaine tubulaire et ayant un organe terminal effecteur qui s'étend de manière distale à partir de l'extrémité distale de la gaine tubulaire.

4. Instrument chirurgical ultrasonore selon la revendication 3, comprenant en outre un bras de préhension monté de manière pivotante sur l'extrémité distale de la gaine tubulaire pour un déplacement en pivotement par rapport à l'organe terminal effecteur de manière à maintenir un tissu entre le bras de préhension et l'organe terminal effecteur.

5. Instrument chirurgical ultrasonore selon l'une quelconque des revendications précédentes, dans lequel ledit segment demi-onde (30a) est associé à d'autres segments demi-onde qui sont sensiblement identiques.
